# EUROPEAN PATENT SPECIFICATION

(11) **EP 0 872 472 B1**
(45) Date of publication and mention of the grant of the patent: **16.08.2001**
(21) Application number: 98106415.7
(22) Date of filing: 07.04.1998
(51) Int. Cl.: C07C 67/54, C07C 69/16

(54) **Process for producing diacetoxybutene**
Verfahren zur Herstellung von Diacetoxybuten
Procédé de préparation du diacétoxybutène

(30) Priority: 16.04.1997 JP 9872697
(43) Date of publication of application: 21.10.1998
(73) Proprietor: Mitsubishi Chemical Corporation, Chiyoda-ku, Tokyo 100 (JP)
(72) Inventor: Kandori, Hiroaki, Mitsubishi Chemical Corporation, Yokkaichi-shi, Mie 510 (JP); Ohkubo, Kazuyuki, Mitsubishi Chemical Corporation, Yokkaichi-shi, Mie 510 (JP); Murai, Nobuyuki, Mitsubishi Chemical Corporation, Yokkaichi-shi, Mie 510 (JP)
(74) Representative: Hansen, Bernd, Dr. Dipl.-Chem.

(56) References cited:
- EP-A- 0 625 503
- DATABASE WPI Week 7751 Derwent Publications Ltd., London, GB; AN 77-90995 XP002067560 & JP 52 133 912 A (MITSUBISHI IND LTD)

## Description

The present invention relates to a process for producing diacetoxybutene. More particularly, this invention relates to an improvement in a process for producing diacetoxybutene by the acetoxylation of butadiene.

Diacetoxybutene is an important intermediate for 1,4-butanediol, used as a starting material for polyurethanes, etc., and for tetrahydrofuran, which is a high-performance solvent.

### BACKGROUND OF THE INVENTION

Diacetoxybutene is produced by reacting butadiene, acetic acid, and oxygen in the presence of a noble-metal catalyst (see, for example, JP-A-52-7907). (The term "JP-A" as used herein means an "unexamined published Japanese patent application".) The diacetoxybutene obtained is hydrogenated using a palladium catalyst or nickel catalyst to obtain diacetoxybutane, which is hydrolyzed to yield 1,4-butanediol (see, for example, JP-A-52-133912).

The above process for producing 1,4-butanediol from butadiene necessitates the use of large amounts of acetic acid and water in the process and yields acetic acid and water as by-products. Various proposals have hence been made on processes for recovering the acetic acid and water for reuse while maintaining a high purity of the aimed product (see the references cited-above). Distillation is usually employed for the separation of the aimed product from acetic acid in these processes.

In these recovery processes, the reaction product mixture resulting from the acetoxylation, i.e., obtained after the reaction of butadiene, acetic acid, and oxygen for yielding diacetoxybutene, is degassed to remove the unreacted butadiene, etc. and then distilled in a distillation column to distill off water and acetic acid through the column top and to discharge residue containing diacetoxybutene from the column bottom. On the other hand, the reaction product obtained by hydrolyzing diacetoxybutane is distilled in a distillation column to distill off water, acetic acid, and other low-boiling substances through the column top and to discharge residue containing 1,4-butanediol from the column bottom.

### SUMMARY OF THE INVENTION

However, when the distillation for recovering acetic acid from the reaction product mixture resulting from the acetoxylation is conducted in a distillation column equipped with an ordinary shell-tube type multitubular heat exchanger as a reboiler, there are often cases where polymer deposition occurs in the reboiler tubes in a relatively short operation period, making it necessary to discontinue the operation. An object of the present invention is to provide an improved method for distilling an acetoxylation reaction product mixture which method can inhibit polymer deposition in reboiler tubes even when the reboiler is operated over a prolonged period of time in the separation by distillation of acetic acid from the reaction product mixture.

The present inventors made intensive studies in order to accomplish the above object. As a result, they have found that the reboiler tubes in a distillation column used for distilling off acetic acid and other substances from the reaction product mixture obtained after acetoxylation can be prevented from being clogged with a polymer deposit over long-term operation by regulating the liquid flow rate in the reboiler tubes. The present invention has been completed based on this finding.

The present invention provides a process for producing diacetoxybutene which comprises reacting butadiene, acetic acid, and oxygen in the presence of a noble-metal catalyst to yield diacetoxybutene and distilling the resultant reaction product mixture containing the diacetoxybutene in a distillation column to distill off low-boiling ingredients such as acetic acid through the column top and to discharge residue containing the diacetoxybutene from the column bottom, and in which the distillation column has a shell-tube type multitubular heat exchanger for heating a column bottom liquid, and the column bottom liquid is passed through the heat exchanger at a flow rate of 0.7 m/sec or higher as measured at the inlets of the heat exchanger tubes. According to this process, heat exchanger clogging by polymer deposition can be avoided and distillation can be continued over a prolonged period of time.

### DETAILED DESCRIPTION OF THE INVENTION

In the process of the present invention, the flow rate of the column bottom liquid passing through the reboiler of a distillation column used for separating acetic acid from an acetoxylation reaction product mixture is regulated to 0.7 m/sec or higher as measured at the reboiler tube inlets. Except this flow rate regulation, the process of the present invention can be suitably carried out according to conventionally known processes for obtaining diacetoxybutene by reacting butadiene, acetic acid, and oxygen.

In the acetoxylation reaction, butadiene, acetic acid, and molecular oxygen are reacted by a known method in the presence of a noble-metal catalyst, in particular a palladium catalyst. The palladium catalyst may consist of palladium metal alone or a salt thereof alone (e.g., an inorganic or organic salt such as palladium chloride, palladium nitrate, or palladium acetate). It is also possible to use a combination of palladium metal or a salt thereof and a promoter comprising a metal, such as bismuth, selenium, antimony, tellurium, or copper, or a compound thereof (e.g., bismuth oxide, selenic acid, tellurium oxide, antimony chloride, or copper chloride). These catalyst components are preferably supported on a carrier such as, e.g., silica, alumina, or active carbon. The catalyst for use in the present invention is usually prepared by impregnating a carrier with a solution containing dissolved therein both a palladium compound and a compound of a promoter ingredient to thereby deposit the catalyst components on the carrier and then reducing the catalyst components on the carrier with hydrogen, methanol, etc. The composition of the catalyst with carrier is usually selected so that the content of palladium therein is from 0.1 to 20% by weight in terms of palladium metal and the content of the promoter therein is from 0.01 to 30% by weight in terms of metals.

The acetoxylation reaction is conducted by any of known processes such as fixed-bed, fluidized-bed, or suspended bed processes. The reaction is carried out at a temperature of generally from 40 to 180°C, preferably from 60 to 150°C, and a pressure of generally from atmospheric pressure to 300 kg/cm² (29.4 MPa), preferably from 30 to 150 kg/cm² (2.94 to 14.7 MPa).

The reaction product mixture thus obtained after the acetoxylation contains unreacted butadiene and other gaseous substances. This reaction mixture therefore is generally degassed to remove the butadiene, etc., before being distilled to obtain diacetoxybutene. The distillation is generally conducted in the following manner. The reaction mixture is fed to a first distillation column to distill off water, acetic acid, monoacetoxybutene, etc. through the column top, and residue discharged from the column bottom is fed to a second distillation column. From the second distillation column, diacetoxybutene is obtained as a distillate, while by-product high-boiling ingredients such as diacetoxyoctadiene are discharged from the column bottom.

The first distillation column usually has theoretical plates of from 4 to 10, and is operated generally under conditions of a column top pressure of from 30 to 300 mmHg (4 to 40 kPa), a column bottom temperature of 190°C or lower, and a reflux ratio of from 0 to 0.1. The second distillation column usually has theoretical plates of from 10 to 15, and is operated generally under the conditions of a column top pressure of from 2 to 100 mmHg (0.3 to 13.3 kPa), a column bottom temperature of 190°C or lower, and a reflux ratio of from 0 to 1.

Each distillation column is usually equipped with, as the reboiler, i.e., as the heater for heating the column bottom liquid, a shell-tube type multitubular heat exchanger in which a heating medium is fed on the shell side and a column bottom liquid to be heated passes within the tubes. In general, the flow of a column bottom liquid through such a heat exchanger is based on natural circulation caused by a difference in specific gravity resulting from heating within the tubes. In rare cases, a heating means such as a thin-film evaporator is employed for, e.g., a distillation column bottom liquid having a high viscosity. The diacetoxybutene obtained by the acetoxylation reaction has a boiling point of about 230°C (101 kPa) and a viscosity of about 2.0 mPa·sec (50°C). It has hence been thought that in designing a reboiler for the first distillation column, special attention need not be paid except that diacetoxybutene should be prevented from polymerizing at high temperatures because it has a double bond.

However, when the present inventors conducted continuous distillation under the aforementioned conditions while keeping the column bottom temperature at 190°C or lower, reboiler tube clogging occurred unexpectedly, making it impossible to continue the distillation over 3,000 to 4,000 hours or more. Although the present inventors investigated the addition of a polymerization inhibitor, e.g., hydroquinone, the addition of a deposition inhibitor, etc. in order to prevent reboiler tube clogging, no effective additive was found.

It was, however, found that polymer deposition on the tubes of a heat exchanger becomes slighter as the flow rate of the column bottom liquid passing through the tubes increases. Polymer deposition is usually diminished significantly when the flow rate of the column bottom liquid is 0.7 m/sec or higher as measured at the tube inlets. In general, the higher the flow rate, the less the polymer deposition. However, increasing the flow rate results in an increased pressure drop in the tubes and thus necessitates a large quantity of energy for circulating the column bottom liquid through the heat exchanger. In addition, there is a fear that an excessively high flow rate may cause tube vibration and lead to a heat exchanger trouble. Consequently, the flow rate of the column bottom liquid passing through the heat exchanger is preferably up to 5.0 m/sec as measured at the tube inlets, and flow rates higher than that are disadvantageous. The flow rate of the column bottom liquid is most preferably from 1.0 to 4.0 m/sec as measured at the tube inlets. The most simple means for passing the column bottom liquid through the heat exchanger at such a flow rate is to dispose a pump in a column bottom liquid conduit extending from the distillation column bottom to the heat exchanger and to feed the column bottom liquid to the heat exchanger with the pump at a flow rate within the specified range. According to the process of the present invention, the first distillation column can be operated continuously throughout about one year by regulating the flow rate of the column bottom liquid passing through the heat exchanger to 0.7 m/sec or higher, especially from 1.0 to 4.0 m/sec, as measured at the tube inlets.

The distillate recovered from the first distillation column through its top, which consists mainly of water and acetic acid, is sent to an acetic acid purification process. The resultant purified acetic acid is reused for the acetoxylation reaction.

The column bottom liquid is sent to the second distillation column, and diacetoxybutene is taken out from the column top according to the method described above. This diacetoxybutene is hydrogenated and then hydrolyzed to yield 1,4-butanediol.

The present invention will be explained below in more detail by reference to Example and Comparative Examples, but the invention should not be construed as being limited thereto unless the invention departs from the spirit thereof.

### EXAMPLE 1

Into an acetoxylation reactor were fed butadiene, acetic acid, and oxygen at rates of 170, 3,000, and 530 parts by weight per hour, respectively. These reactants were reacted at 9 MPa and 100°C in the presence of a catalyst comprising 3% by weight palladium and 0.6% by weight tellurium both supported on active carbon. The resultant reaction mixture was discharged from the reactor and then degassed to thereby obtain a reaction product mixture containing 14.2% by weight diacetoxybutene.

The reaction product mixture was fed to a first distillation column at a rate of 3,100 parts by weight per hour and was distilled therein to distill off acetic acid. The distillation column used was a packed column having a theoretical plates of 10. The reaction product mixture was fed on the eighth plate from the column top. The distillation column was operated under the conditions of a column top pressure of 13.3 kPa (100 mmHg), a reflux ratio of 0.1, and a column bottom temperature of 150°C. The column bottom liquid was circulated through the reboiler at a rate of 1.5 m/sec as measured at the reboiler tube inlets, by means of a pump disposed between the bottom of the distillation column and the reboiler. The water and acetic acid contained in the reaction product mixture were mostly distilled off through the column top, and a column bottom liquid containing 84.5% by weight diacetoxybutene and 3.2% by weight diacetoxyoctadiene was discharged from the column bottom at a rate of 580 parts by weight per hour. The distillation column was continuously operated for about one year (8,000 hours) under the above conditions. Thereafter, the reboiler was disassembled to inspect the tubes thereof. As a result, about 2% of all the tubes were found to have been clogged. At the time when distillation operation started, the reboiler had an overall coefficient of heat transmission of 780 kcal/hr·m²·°C.

### COMPARATIVE EXAMPLE 1

The same distillation column as in Example 1 was operated under the same conditions as in Example 1, except that the pump was not used and the column bottom liquid was allowed to pass through the reboiler based on natural circulation. The reboiler had been designed to have a flow rate as measured at the tube inlets of 0.3 m/sec under these conditions. The vapor pressure in the reboiler on the shell side began to increase at the time when about 2,000 hours had passed since the initiation of distillation operation, and the operation became impossible in about 3,000 hours. The reboiler was disassembled and inspected. As a result, about 40% of all the tubes were found to have been clogged with a polymer deposit. At the time when distillation operation started, the reboiler had an overall coefficient of heat transmission of 380 kcal/hr·m²·°C.

### COMPARATIVE EXAMPLE 2

The same distillation column as in Example 1 was operated under the same conditions as in Example 1, except that the column bottom liquid was fed to the reboiler with the pump at a flow rate of 0.5 m/sec as measured at the reboiler tube inlets.

The vapor pressure in the reboiler on the shell side began to increase at the time when about 3,000 hours had passed since the initiation of distillation operation, and the operation became impossible in about 4,000 hours. The reboiler was disassembled and inspected. As a result, about 40% of all the tubes were found to have been clogged with a polymer deposit. At the time when distillation operation started, the reboiler had an overall coefficient of heat transmission of 450 kcal/hr·m²·°C.

According to the present invention, the separation of acetic acid from a reaction product mixture resulting from acetoxylation for yielding diacetoxybutene from butadiene using a noble-metal catalyst can be stably conducted in a distillation column over a prolonged period of time while preventing the reboiler of the distillation column from suffering tube clogging.

## Claims

1. A process for producing diacetoxybutene which comprises reacting butadiene, acetic acid, and oxygen in the presence of a noble-metal catalyst to yield diacetoxybutene and distilling the resultant reaction product mixture containing the diacetoxybutene in a distillation column to distill off acetic acid and other low-boiling ingredients through the column top and to discharge residue containing the diacetoxybutene from the column bottom, said distillation column having a shell-tube type multitubular heat exchanger for heating a column bottom liquid, the column bottom liquid being passed through the heat exchanger at a flow rate of 0.7 m/sec or higher as measured at the inlets of the heat exchanger tubes.

2. The process of claim 1, wherein the column bottom liquid is passed through the heat exchanger at a flow rate of from 1.0 to 4.0 m/sec as measured at the inlets of the heat exchanger tubes.

3. The process of claim 1, wherein the column bottom liquid is fed to the heat exchanger by means of a pump.

4. The process of claim 1, wherein the reaction product mixture to be subjected to distillation in the distillation column is one obtained by degassing the reaction mixture obtained by reacting butadiene, acetic acid, and oxygen in the presence of a noble-metal catalyst.

5. The process of claim 1, wherein the noble-metal catalyst comprises a carrier having supported thereon, palladium and at least one member selected from the group consisting of bismuth, selenium, antimony, tellurium, and copper.

6. The process of claim 1, wherein the temperature of the column bottom liquid in the distillation column is kept at 190°C or lower.

7. The process of claim 1, wherein the reaction product mixture obtained by reacting butadiene, acetic acid and oxygen in the presence of a catalyst comprising palladium supported on a carrier is fed to the distillation column after degassing, and the column bottom liquid is passed through the heat exchanger at a flow rate of 1.0 to 4.0 m/sec as measured at the inlets of the heat exchanger tubes while the temperature of the liquid being kept at 190°C or lower.

## Patentansprüche

1. Verfahren zur Herstellung von Diacetoxybuten, umfassend Umsetzen von Butadien, Essigsäure und Sauerstoff in Gegenwart eines Edelmetall-Katalysators unter Erhalt von Diacetoxybuten und Destillieren des resultierenden Reaktionsproduktgemisches, das das Diacetoxybuten enthält, in einer Destillationskolonne, wobei Essigsäure und andere niedrigsiedende Ingredienzien durch den oberen Teil der Kolonne abdestilliert werden und der Rückstand, der das Diacetoxybuten enthält, aus dem Kolonnenboden entnommen wird, wobei die Destillationskolonne einen Röhren-Wärmeaustauscher des Rohrbündel-Typs zum Erwärmen einer Kolonnenbodenflüssigkeit hat, die Kolonnenbodenflüssigkeit mit einer Strömungsgeschwindigkeit von 0,7 m/s oder höher, gemessen an den Einlaßöffnungen oder Wärmeaustauscherröhren, durch den Wärmeaustauscher geführt wird.

2. Verfahren nach Anspruch 1, wobei die Kolonnenbodenflüssigkeit mit einer Strömungsgeschwindigkeit von 1,0 bis 4,0 m/s, gemessen an den Einlaßöffnungen der Wärmeaustauscherröhren, durch den Wärmeaustauscher geführt wird.

3. Verfahren nach Anspruch 1, wobei die Kolonnenbodenflüssigkeit dem Wärmeaustauscher mit Hilfe einer Pumpe zugeführt wird.

4. Verfahren nach Anspruch 1, wobei das Reaktionsproduktgemisch, das einer Destillation in der Destillationskolonne unterworfen werden soll, ein Reaktionsproduktgemisch ist, das durch Entgasen des Reaktionsgemisches erhalten wird, welches durch Umsetzen von Butadien, Essigsäure und Sauerstoff in Gegenwart eines Edelmetall-Katalysators erhalten wird.

5. Verfahren nach Anspruch 1, wobei der Edelmetall-Katalysator einen Träger, der Palladium und mindestens ein Element, das aus der Gruppe, bestehend aus Wismut, Selen, Antimon, Tellur und Kupfer ausgewählt wird, darauf aufgetragen hat, umfaßt.

6. Verfahren nach Anspruch 1, wobei die Temperatur der Kolonnenbodenflüssigkeit in der Destillationskolonne bei 190°C oder niedriger gehalten wird.

7. Verfahren nach Anspruch 1, wobei das Reaktionsproduktgemisch, das durch Umsetzen von Butadien, Essigsäure und Sauerstoff in Gegenwart eines Katalysators, der Palladium auf einem Träger umfaßt, erhalten wird, nach einem Entgasen zu der Destillationskolonne geführt wird und die Kolonnenbodenflüssigkeit mit einer Strömungsgeschwindigkeit von 1,0 bis 4,0 m/s, gemessen an den Einlaßöffnungen der Wärmeaustauscherröhren, durch den Wärmeaustauscher geführt wird, während die Temperatur und der Flüssigkeit bei 190°C oder niedriger gehalten wird.

## Revendications

1. Procédé pour produire du diacétoxybutène qui comprend de faire réagir du butadiène, de l'acide acétique et de l'oxygène en présence d'un catalyseur de métal noble pour obtenir du diacétoxybutène et de distiller le mélange des produits de la réaction résultant contenant le diacétoxybutène dans une colonne de distillation pour éliminer par distillation l'acide acétique et d'autres composants à bas point d'ébullition par le haut de la colonne et pour décharger un résidu contenant le diacétoxybutène depuis le bas de la colonne, ladite colonne de distillation ayant un échangeur thermique multitubulaire de type tube à enveloppe pour chauffer un liquide au bas de la colonne, le liquide au bas de la colonne étant envoyé à travers l'échangeur thermique avec un débit de 0,7 m / sec ou plus, la mesure étant faite aux entrées des tubes de l'échangeur thermique.

2. Procédé selon la revendication 1, dans lequel le liquide au bas de la colonne est envoyé à travers l'échangeur thermique avec un débit de 1,0 à 4,0 m/sec, la mesure étant faite aux entrées des tubes de l'échangeur thermique.

3. Procédé selon la revendication 1, dans lequel le liquide au bas de la colonne est amené à l'échangeur thermique au moyen d'une pompe.

4. Procédé selon la revendication 1, dans lequel le mélange des produits de la réaction devant être soumis à une distillation dans la colonne de distillation est un mélange obtenu par le dégazage du mélange réactionnel obtenu en faisant réagir du butadiène, de l'acide acétique et de l'oxygène en présence d'un catalyseur de métal noble.

5. Procédé selon la revendication 1, dans lequel le catalyseur de métal noble comprend un support portant du palladium et au moins un élément choisi dans le groupe constitué par le bismuth, le sélénium, l'antimoine, le tellure et le cuivre.

6. Procédé selon la revendication 1, dans lequel la température du liquide au bas de la colonne dans la colonne de distillation est maintenue à 190 °C ou plus bas.

7. Procédé selon la revendication 1, dans lequel le mélange des produits de la réaction obtenu en faisant réagir du butadiène, de l'acide acétique et de l'oxygène en présence d'un catalyseur comprenant du palladium fixé sur un support est introduit dans la colonne de distillation après dégazage, et le liquide au bas de la colonne est envoyé à travers l'échangeur thermique avec un débit de 1,0 à 4,0 m / sec, la mesure étant faite aux entrées des tubes de l'échangeur thermique, alors que la température du liquide est maintenue à 190 °C ou plus bas.
